(19) **Europäisches Patentamt / European Patent Office / Office européen des brevets**

(11) **EP 4 065 222 B1**

(12) **FASCICULE DE BREVET EUROPEEN**

(45) Date de publication et mention de la délivrance du brevet:
**18.02.2026   Bulletin 2026/08**

(21) Numéro de dépôt: **20808460.8**

(22) Date de dépôt: **25.11.2020**

(51) Classification Internationale des Brevets (IPC):
**A61N 7/00** *(2006.01)*       **A61B 8/08** *(2006.01)*

(52) Classification Coopérative des Brevets (CPC):
**A61N 7/00;** A61N 2007/0021; A61N 2007/0052;
A61N 2007/0078; A61N 2007/0082

(86) Numéro de dépôt international:
**PCT/EP2020/083313**

(87) Numéro de publication internationale:
**WO 2021/105179 (03.06.2021 Gazette 2021/22)**

(54) **DISPOSITIF MEDICAL IMPLANTABLE POUR L'IMAGERIE ET/OU LE TRAITEMENT D'UN TISSU CEREBRAL**

IMPLANTIERBARE MEDIZINISCHE VORRICHTUNG ZUR ABBILDUNG UND/ODER BEHANDLUNG VON HIRNGEWEBE

IMPLANTABLE MEDICAL DEVICE FOR IMAGING AND/OR TREATMENT OF BRAIN TISSUE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:   **25.11.2019   FR 1913189**

(43) Date de publication de la demande:
**05.10.2022   Bulletin 2022/40**

(73) Titulaire: **Carthera**
**69008 Lyon (FR)**

(72) Inventeurs:
 • **VIGNOT, Alexandre**
   **69003 LYON (FR)**

 • **CHOLVY, Matthieu**
   **38550 PEAGE DE ROUSSILLON (FR)**

(74) Mandataire: **Be IP**
**Cabinet LTL SAS**
**Centre d'Entreprise et d'Innovation**
**56, Bd Niels Bohr**
**CS 52132**
**69603 Villeurbanne Cedex (FR)**

(56) Documents cités:
**EP-A2- 3 233 187       FR-A1- 3 078 879
US-A1- 2009 112 278**

**Description**

## DOMAINE DE L'INVENTION

La présente invention concerne le domaine technique général des dispositifs ultrasonores pour le traitement d'un tissu cérébral - humain ou animal - par ultrasons afin d'aider un praticien dans le traitement d'une pathologie.

## ARRIERE PLAN DE L'INVENTION

On connaît différentes techniques permettant de traiter un tissu cérébral.

FR 3 078 879 A1 décrit un dispositif implantable au niveau d'une ouverture ménagée dans la boîte crânienne d'un patient, comprenant une plaque support et une unité ultrasonore.

Notamment, une technique connue développée par la Demanderesse consiste à utiliser un appareil de traitement comprenant :

- un dispositif intracrânien implantable,
- une unité de commande distante du dispositif intracrânien, et
- des moyens de connexion entre le dispositif intracrânien et l'unité de commande.

Le dispositif intracrânien est destiné à être positionné dans un trou de trépan effectué dans un crâne d'un patient. Il comprend :

- un support,
- un (ou plusieurs) transducteur(s) pour la génération d'ondes ultrasonores de traitement, monté(s) sur le support,
- une (ou plusieurs) borne(s) de connexion électrique destinée(s) à coopérer avec les moyens de connexion.

L'unité de commande 2 est apte à alimenter en énergie électrique le dispositif intracrânien, et à régler ses paramètres de fonctionnement.

Les moyens de connexion sont adaptés pour relier électriquement le dispositif intracrânien à l'unité de commande. Ils comprennent généralement :

- un (ou plusieurs) câble(s) de connexion électrique dont l'une des extrémités est reliée à l'unité de commande, et
- une (ou plusieurs) aiguille(s) transdermique(s) raccordée(s) à l'autre extrémité du câble.

Le principe de fonctionnement de cet appareil est le suivant. Une fois le dispositif intracrânien implanté dans le crâne du patient, une succession de séances de traitement lui est prodiguée pour traiter la pathologie qui l'affecte.

A chaque nouvelle séance de traitement, le dispositif intracrânien est relié à l'unité de commande par l'intermédiaire des moyens de connexion : le praticien relie le câble à l'unité de commande puis il insère l'aiguille à travers la peau du patient jusqu'à la borne du dispositif ultrasonore.

Une fois l'extrémité de l'aiguille connectée à la borne, l'unité de commande peut être activée pour alimenter le dispositif ultrasonore en énergie électrique.

La technique décrite ci-dessus permet un traitement efficace des affections du cerveau.

Un but de la présente invention est de proposer un dispositif intracrânien amélioré permettant d'augmenter la sécurité du patient lors de la phase de raccordement du dispositif intracrânien à l'unité de commande, en particulier lors de l'insertion de l'aiguille à travers la peau du crâne du patient.

Un autre but de la présente invention est de proposer un dispositif intracrânien amélioré dont les risques de détérioration sont réduits lors de la phase de raccordement du dispositif intracrânien à l'unité de commande, en particulier lors de l'insertion de l'aiguille à travers la peau du crâne du patient.

## BREVE DESCRIPTION DE L'INVENTION

A cet effet, l'invention propose un dispositif implantable au niveau d'une ouverture ménagée dans la boîte crânienne d'un patient, le dispositif implantable comprenant :

- une plaque support incluant une première face et une deuxième face opposée à la première face,
- une unité ultrasonore destinée à être montée sur la plaque support, ladite unité ultrasonore comprenant :

    o un logement incluant un fond, au moins une paroi latérale et une face supérieure opposée au fond, le logement étant destiné à être positionné sur la première face de la plaque support,
    o une pluralité de transducteurs, disposés dans le logement, pour la génération d'ondes ultrasonores de traitement d'une affection cérébrale,
    o une borne de connexion électrique, fixée au logement, pour le raccordement de l'unité ultrasonore à une unité de commande distante par l'intermédiaire de moyens de connexion électrique,

- la borne de connexion comprenant un pion s'étendant en saillie vers l'extérieur du logement dans une direction perpendiculaire au fond et d'orientation allant du fond vers la face supérieure du logement,
- la plaque support comprenant un orifice traversant pour le passage du pior remarquable en ce que:

    - le dispositif implantable comprend en outre une pièce de fixation incluant :

o un conduit adapté pour recevoir au moins une portion du pion, et

o une collerette périphérique s'étendant perpendiculairement à un axe longitudinal du conduit,

la collerette étant destinée à venir en contact avec la deuxième face de la plaque support pour plaquer la plaque support contre le logement lorsque l'unité ultrasonore, la plaque support et la pièce de fixation sont assemblées.

Le fait que l'unité ultrasonore, la plaque support et la pièce de fixation consistent en trois composants indépendants permet de faciliter la fabrication du dispositif implantable, chacun de ces éléments pouvant être fabriqué séparément.

Le fait que :

- l'unité ultrasonore comprenne une borne de connexion s'étendant en saillie vers l'extérieur, que
- la plaque support comprenne un orifice traversant pour le passage de la borne de connexion et que
- la pièce de fixation soient destinée à coopérer avec la borne de connexion une fois celle-ci insérée dans l'orifice traversant pour appuyer la plaque support contre l'unité ultrasonore

permet de faciliter l'assemblage des différents éléments constituant le dispositif implantable, et donc son procédé de fabrication.

Le fait que la pièce de fixation comprenne une collerette permet :

- d'une part de protéger la carte électronique contenue dans le logement de l'unité ultrasonore, notamment dans le cas où le praticien insèrerait l'aiguille transdermique à une position voisine du trou borgne destiné à recevoir l'aiguille,
- d'autre part de limiter la déformation de la plaque support en répartissant la force appliqué par l'aiguille transdermique (lors de l'insertion de celle-ci dans la borne de connexion) sur une grande surface de la plaque support.

Des aspects préférés mais non limitatifs de la présente invention sont les suivants :

- le rapport entre la largeur de la collerette $L_{Coll}$ et la largeur du conduit $L_{Cond}$ peut être compris entre ¼ et 2, préférentiellement ½ et $^3/_2$, et encore plus préférentiellement égal à 1 ;
- le rapport entre la largeur de la collerette $L_{Coll}$ et la largeur de la face supérieure $L_{Log}$ du logement peut être compris entre ½ et 2, et préférentiellement entre $^2/_3$ et 1 ;
- la collerette peut être de forme circulaire ;
- la collerette peut comprendre au moins deux lumières traversantes diamétralement opposées ;

- le pion peut être de forme cylindrique et comprendre un filetage sur sa paroi latérale, le conduit consistant en un écrou dont le trou taraudé est destiné à coopérer par vissage avec le filetage de la paroi latérale du pion ;
- le pion peut comprendre une paroi supérieure opposée au fond du logement, et un trou borgne destiné à recevoir une extrémité des moyens de connexion, la paroi supérieure incluant une fraisure ménagée à l'entrée du trou borgne ;
- le pion peut comprendre une butée à sa base, le bord de l'orifice traversant de la plaque support étant destiné à venir en appui contre la butée lorsque le pion est inséré dans ledit orifice traversant ;
- le bord de l'orifice traversant de la plaque support peut être recouvert d'une couche de matériau polymère, tel que de la silicone ;
- la distance entre le centre de l'orifice traversant et le barycentre de la plaque peut être non nulle.

L'invention concerne également un système pour l'imagerie et/ou le traitement d'un tissu cérébral, le système incluant une unité de commande et des moyens de connexion électrique, remarquable en ce que le système comprend en outre un dispositif implantable tel que décrit ci-dessus.

## BREVE DESCRIPTION DES DESSINS

D'autres avantages et caractéristiques de l'invention ressortiront mieux de la description qui va suivre de plusieurs variantes d'exécution, données à titre d'exemples non limitatifs, à partir des dessins annexés sur lesquels :

- La figure 1 est une représentation schématique en perspective d'un dispositif médical implantable,
- La figure 2 est une représentation schématique en perspective d'une unité ultrasonore du dispositif médical implantable,
- La figure 3 est une représentation schématique en perspective d'une plaque support du dispositif médical implantable,
- La figure 4 est une représentation schématique en perspective d'une pièce de fixation du dispositif médical implantable.

## DESCRIPTION DETAILLEE DE L'INVENTION

On va maintenant décrire un exemple de dispositif médical implantable en référence aux figures. Dans ces différentes figures, les éléments équivalents sont désignés par la même référence numérique.

### 1. Généralités

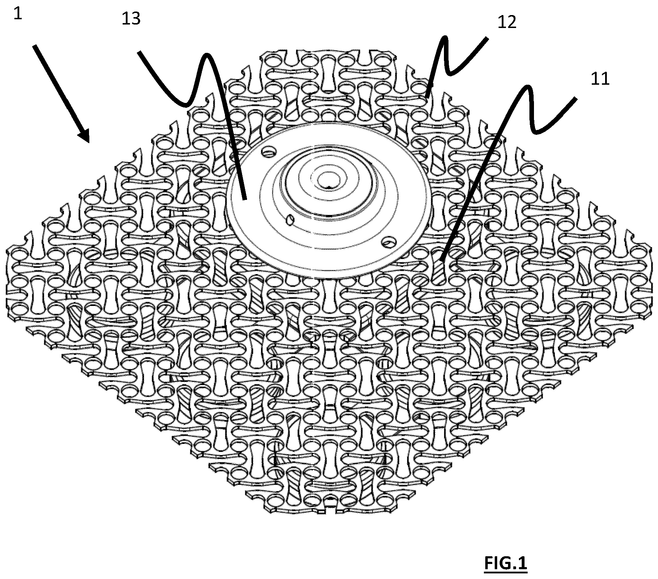

En référence à la figure 1, le dispositif médical implantable 1 comprend :

- une unité ultrasonore 11 pour l'émission d'ondes ultrasonores d'imagerie ou de traitement,
- une plaque support 12 sur laquelle est montée l'unité ultrasonore 11, et
- une pièce de fixation 13 pour bloquer l'unité ultrasonore 11 contre la plaque support 12.

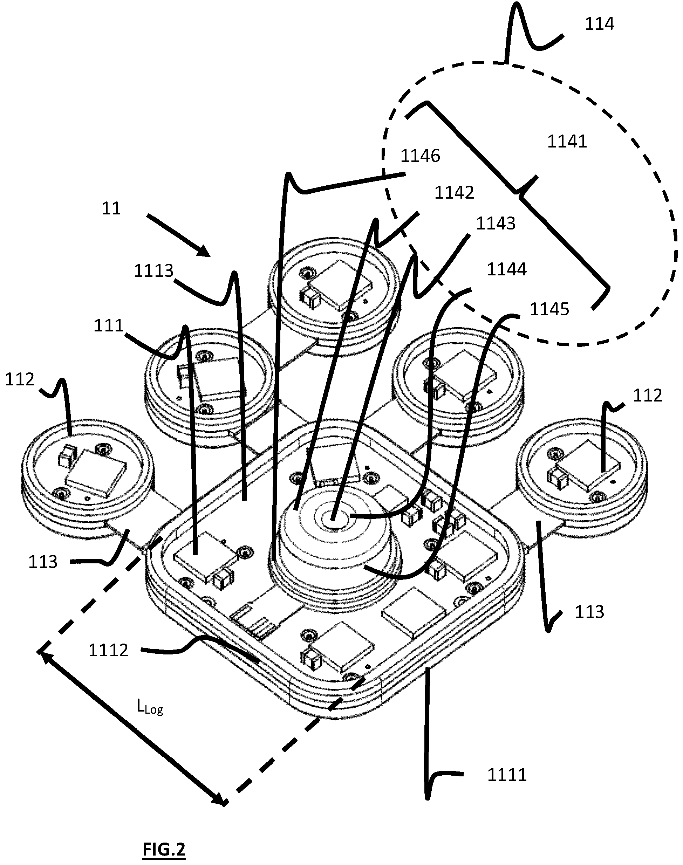

Comme il ressort des figures 2 à 4, l'unité ultrasonore 11, la plaque support 12, et la pièce de fixation 13 sont des éléments distincts destinés à être assemblés pour former le dispositif médical implantable 1. Plus précisément pour former le dispositif médical implantable 1, ces différents éléments sont assemblés de sorte que la plaque support 12 s'étende entre l'unité ultrasonore 11 et la pièce de fixation 13.

Le dispositif médical 1 est apte à être implanté dans un os crânien d'un patient pour permettre le traitement et/ou l'imagerie d'une zone cérébrale d'intérêt.

Pour ce faire, le praticien réalise une craniectomie. Une incision est faite dans le cuir chevelu, puis la peau (et les muscles le cas échéant) est soulevée (sont soulevés) afin d'exposer le crâne. Le crâne est ensuite découpé pour former un volet osseux. Le volet osseux crânien est retiré pour laisser place à une ouverture crânienne dans laquelle le dispositif intracrânien peut être positionné.

Le positionnement du dispositif 1 consiste à l'insérer dans l'ouverture crânienne de sorte que l'unité ultrasonore 11 s'étende en regard de la zone cérébrale d'intérêt. Une fois le dispositif 1 correctement positionné, la bordure 124 de la plaque support 12 est fixée sur la périphérie de l'ouverture crânienne par tout moyen connu de l'homme du métier (vis d'ancrage, collage, etc.), puis le cuir chevelu et les muscles sont remis en place pour recouvrir le dispositif 1. Ainsi, une fois implanté, l'unité ultrasonore 11 fait face à la zone cérébrale d'intérêt, tandis que la pièce de fixation 13 s'étend en regard du cuir chevelu du patient.

A chaque nouvelle séance de traitement, le praticien raccorde électriquement le dispositif médical 1 à l'unité de commande distante en utilisant les moyens de connexion. Ces moyens de connexion comprennent notamment :

- un câble électriquement conducteur,
- une aiguille montée à l'une des extrémités du câble, l'aiguille étant apte à être introduite dans une borne de connexion de l'unité ultrasonore, et
- une prise de liaison à l'autre des extrémités du câble, la prise de liaison étant apte à être connectée à une prise complémentaire de l'unité de commande.

Plus précisément, le praticien connecte la prise de liaison à l'unité de commande distante. Le praticien insère ensuite l'aiguille dans le cuir chevelu du patient, et introduit l'extrémité de l'aiguille dans un trou borgne de la borne de connexion de sorte à finaliser le raccordement électrique du dispositif ultrasonore 1 à l'unité de commande distante.

On va maintenant décrire plus en détails les différents éléments (unité ultrasonore, plaque support, pièce de fixation) constituant le dispositif médical implantable.

## 2. *Unité ultrasonore*

En référence à la figure 2, l'unité ultrasonore 11 comprend :

- un logement 111,
- une borne de connexion électrique 114 pour le raccordement de l'unité ultrasonore 11 à une unité de commande distante.

### 2.1. *Logement*

Le logement 111 comporte un fond 1111 et (une ou) plusieurs parois latérales 1112. Dans le mode de réalisation illustré à la figure 2, la face supérieure du logement 111 opposée au fond 1111 est ouverte. En d'autres termes, la face supérieure du logement est dépourvue de paroi supérieure. Ceci permet de favoriser la dissipation vers l'extérieur de la chaleur générée par les cartes électroniques. De préférence, la face supérieure du logement 111 est recouverte de parylène qui présente l'avantage d'être biocompatible.

Ce logement 111 est positionné sur une première face de la plaque support 12 lorsque le dispositif médical est assemblé.

Le logement 111 est prévu pour recevoir :

- une carte électronique principale 1113 adaptée pour échanger des signaux électriques d'alimentation et de contrôle avec l'unité de commande distante, et
- des transducteurs ultrasonores - par exemple circulaires de 10 millimètres de diamètre chacun - adaptés pour générer des ondes ultrasonores de traitement (ou d'imagerie) de la zone cérébrale d'intérêt.

La carte électronique 1113 et les transducteurs étant connus de l'homme du métier, ceux-ci ne seront pas décrits plus en détails dans la suite.

Le lecteur appréciera néanmoins qu'outre le logement 111, l'unité ultrasonore 11 peut comprendre des réceptacles secondaires 112 incluant chacun une carte électronique secondaire et un (ou plusieurs) transducteur(s) additionnel(s).

Le logement 111 et les réceptacles secondaires 112 sont répartis pour former un réseau de transducteurs régulièrement espacés. Les cartes électroniques secondaires peuvent être reliées à la carte électronique principales 1113 par l'intermédiaire de connectiques flexo-rigides 113 pour le transfert de signaux d'alimentation et de contrôle issus de l'unité de commande externe (non représentée). Comme illustré à la figure 1, le logement 111 et les réceptacles 112 s'étendent sous la plaque support 12 dont les dimensions sont choisies de sorte à recouvrir l'unité ultrasonore 11, la plaque support étant destinée à être fixée sur le crâne du patient au niveau de ses bords.

### 2.2. *Borne de connexion*

La borne de connexion électrique 114 est fixée au logement 111. Elle permet de connecter le dispositif médical 1 à l'unité de commande externe qui alimente les transducteurs en énergie électrique, et règle leurs paramètres de fonctionnement.

Comme indiqué précédemment, la borne de connexion est adaptée pour coopérer avec l'aiguille transdermique des moyens de connexion.

Plus précisément, la borne de connexion 114 comprend un pion 1141 s'étendant en saillie vers l'extérieur du logement 111 dans une direction perpendiculaire au fond 1111 et d'orientation allant du fond 1111 vers la face supérieure du logement 111.

La base du pion comprend de préférence une butée 1146 sur laquelle la plaque support 12 est destinée à venir en appui. Ceci permet de maintenir une distance non nulle entre le logement 111 de l'unité ultrasonore 11 et la plaque support 12 afin de limiter les contacts entre le logement 111 et la plaque support 12.Ceci permet d'éviter d'endommager la couche de parylène recouvrant le logement 111 au niveau de sa face supérieure.

La paroi supérieure 1142 du pion 1141 comporte un trou borgne 1143 dans lequel l'extrémité de l'aiguille des moyens de connexion est destinée à être introduite pour raccorder électriquement l'unité ultrasonore 11 aux moyens de connexion électrique.

Avantageusement, la borne de connexion 114 peut comprendre un évasement conique (ou fraisure) 1144 ménagé à l'entrée du trou borgne 1143. Ceci permet de faciliter l'introduction de l'extrémité de l'aiguille dans le trou borgne 1143. En effet, comme il est rappelé ci-dessus, la borne de connexion 114 est recouverte par la peau du crâne. Il peut donc s'avérer difficile pour le praticien d'introduire l'extrémité de l'aiguille dans le trou borgne 1143 lors de la phase de raccordement électrique du dispositif médical 1 à l'unité de commande. La présence d'une fraisure 1144 permet de faciliter cette opération de raccordement, l'évasement conique permettant de guider l'aiguille vers le trou borgne 1143 lorsque l'extrémité de l'aiguille est à proximité du trou borgne 1143.

Comme illustré à la figure 2, la paroi latérale du pion 1141 peut comprendre un filetage 1145. Ce filetage est destiné à coopérer par vissage avec un filetage correspondant ménagé sur la face interne d'un conduit de la pièce de fixation. Ceci permet d'assurer la solidarisation de l'unité ultrasonore 11, de la plaque support 12 et de la pièce de fixation 13 lors de l'assemblage du dispositif médical 1. Le fait que l'unité ultrasonore 11 coopère par vissage avec la pièce de fixation 13 permet, lors de la lors de la phase de raccordement du dispositif intracrânien à l'unité de commande, de répartir la force appliquée par l'aiguille sur la borne de connexion à toute une surface de la plaque support 12.

### 3. *Plaque support*

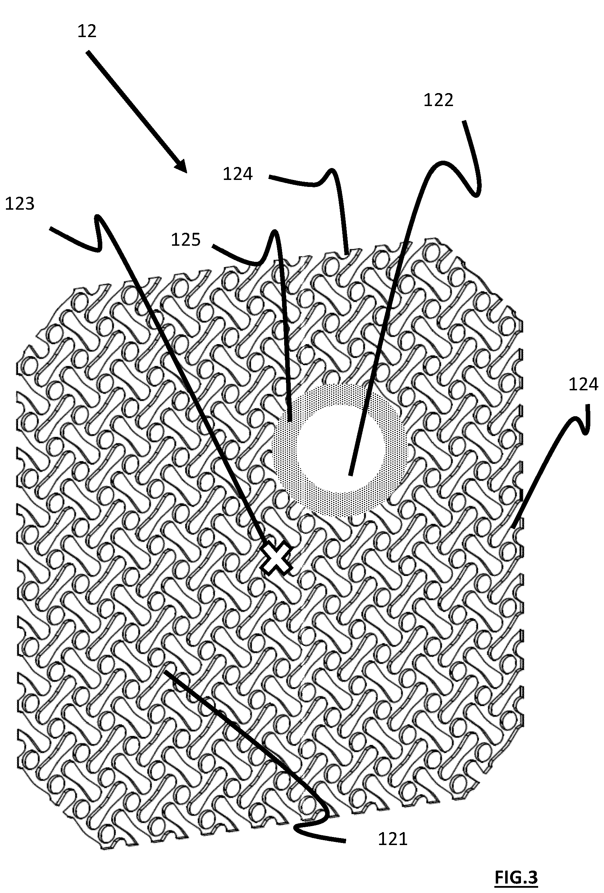

En référence à la figure 3, on a illustré un exemple de plaque support 12.

La plaque support 12 est généralement rectangulaire, mais peut présenter une forme quelconque, telle qu'une forme circulaire, triangulaire ou carrée. Les dimensions de la plaque support 12 (longueur et largeur) peuvent être comprises entre 1 et 15 centimètres.

La plaque support 12 peut être sensiblement plane. En variante, la plaque support 12 peut être incurvée ou déformée pour suivre la courbure de la boîte crânienne du patient.

Dans le mode de réalisation illustré à la figure 3, la plaque support 12 comprend une pluralité d'ouvertures traversantes 121. Ces ouvertures traversantes peuvent être de formes différentes (ronde, triangulaire, elliptique, pentagonale, hexagonal, en nid d'abeille, en losange, etc.). Les ouvertures traversantes 121 peuvent être obtenues par perçage d'une plaque pleine, par découpe chimique, par moulage, ou par tissage de fils - notamment métalliques - afin de former un grillage constitué de fils maillés. La présence d'ouvertures traversantes dans la plaque support permet de faciliter la déformation de la plaque support afin de conformer celle-ci à la forme du crâne du patient. Dans d'autres modes de réalisation, la plaque support 12 peut être une plaque pleine dépourvue d'ouvertures traversantes 121.

Dans tous les cas, la plaque support 12 comprend un orifice traversant 122 pour le passage du pion de la borne de connexion. Comme illustré à la figure 3, l'orifice traversant 122 est décalé relativement au barycentre 123 de la plaque support. Plus précisément, l'orifice traversant 122 est ménagé dans la plaque support 12 de sorte à s'étendre à proximité de l'une (ou de plusieurs) des bordures 124 de la plaque support 12. Ceci permet de limiter les risques de déformation mécanique de la plaque support 12 lors de l'insertion de l'aiguille dans la borne de connexion pour raccorder électriquement le dispositif médical implantable à l'unité de commande distante.

Le bord 125 de l'orifice traversant 122 peut être recouvert d'une couche de matériau polymère, tel que de la silicone. Dans ce cas, la couche de matériau polymère est déposée sur le bord 125 lors de l'assemblage du dispositif médical implantable 1. Cette couche de matériau polymère permet de limiter les risques de desserrage entre la borne de connexion 114 et la pièce de fixation 13.

Le matériau constituant la plaque support 12 peut être un métal, tel que du titane ou tout autre métal connu de l'homme du métier (éventuellement recouvert de parylène ou équivalent si le métal utilisé n'est pas biocompatible en soi). L'utilisation d'une plaque en métal permet de limiter sa déformation en réponse à l'application d'une force d'appui (par exemple déformation mécanique inférieure à 2,5mm en réponse à une force d'appui de 50 Newtons appliquée au centre de la plaque). L'utilisation de titane présente quant à elle de nombreux avantages, le titane étant un matériau très solide et bien accepté par la structure osseuse.

### 4. *Pièce de fixation*

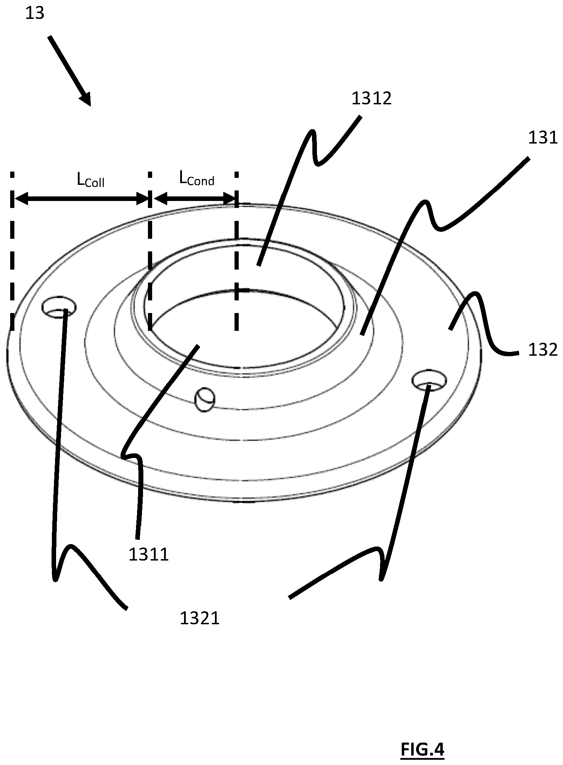

En référence à la figure 4, la pièce de fixation 13 comprend :

- un conduit 131 apte à recevoir le pion 1141, et
- une collerette 132 périphérique.

Le conduit est destiné à coopérer avec la borne de connexion de sorte à bloquer la plaque support entre l'unité ultrasonore et la pièce de fixation. Plus précisément, le conduit consiste en un écrou dont le trou taraudé est destiné à coopérer par vissage avec le filetage 1145 de la paroi latérale du pion 1141. En d'autres termes, la face interne du conduit 131 comprend un filet 1312 complémentaire du filetage 1145 de la paroi latérale du pion 1141.

La collerette 132 s'étend à la base du conduit 131, perpendiculairement à l'axe de révolution du conduit 131. Elle est destinée à venir en contact avec une deuxième face de la plaque support 12 opposée à la première face en regard du logement 111. La collerette 132 permet de plaquer la plaque support 12 contre la butée 1146 lorsque l'unité ultrasonore 11, la plaque support 12 et la pièce de fixation 13 sont assemblées.

Le rapport entre la largeur $L_{Coll}$ de la collerette 132 et la largeur $L_{Cond}$ du conduit peut être compris entre 1/4 et 2, préférentiellement ½ et 3/2, et encore plus préférentiellement égal à 1. Plus précisément et en référence à la figure 1, les dimensions de la collerette 132 sont choisies suffisantes pour que l'ensemble composé de la surface couverte par le conduit et de la surface de la collerette 132 recouvre en quasi-totalité (i.e. au moins 2/3 de) la face supérieure du logement 111. Notamment, le rapport entre la largeur $L_{Coll}$ de la collerette 132 et la largeur $L_{Log}$ de la face supérieure du logement 111 (i.e. diamètre dans le cas d'une collerette annulaire et/ou d'une face supérieure de logement circulaire, diagonale entre deux sommets opposés dans le cas d'une collerette parallélépipédique et/ou d'une face supérieure de logement parallélépipédique, etc.) est compris entre ½ et 2, et préférentiellement entre 2/3 et 1. Ceci permet de disposer d'une collerette 132 de surface suffisante pour protéger la carte électronique 1113 d'éventuels dommages causés par l'extrémité de l'aiguille, dans l'hypothèse où le praticien insèrerait celle-ci à une position décalée par rapport au trou borgne 1143.

De préférence, la collerette 132 est de forme circulaire. Ceci permet une meilleure répartition de la force appliquée par l'aiguille lors de l'introduction de celle-ci dans le trou borgne 1143 de la borne de connexion114.

Avantageusement, la collerette 132 peut comprendre deux (ou plus de deux) lumières traversantes 1321 diamétralement opposées. Ces lumières 1321 permettent de faciliter le vissage de la pièce de fixation 13 sur le pion 1141. Ces lumières permettent en outre d'appliquer, en fabrication, un couple de serrage prédéfini pour éviter toute perte d'étanchéité au liquide du dispositif implantable. En effet, le pion 1141 est réalisé en deux parties (une première partie montée sous le fond du logement et une deuxième partie montée sur le fond, un joint d'étanchéité (qu'il faut comprimer au montage) étant disposé entre les deux parties).

Le principe d'assemblage du dispositif implantable 1 est le suivant. L'opérateur insère le pion 1141 de la borne de connexion 114 à travers l'orifice traversant 122 de la plaque support 12. Une fois l'unité ultrasonore 11 en position sur la première face de la plaque support 12, l'opérateur dépose la couche de matériau polymère (par exemple silicone) sur la plaque support, en particulier au niveau du bord 125 de l'orifice traversant 122. L'opérateur dispose ensuite la pièce de fixation 13 sur la borne de connexion 114. La pièce de fixation 13 est installée sur le pion 1141 de sorte que la base du conduit 131 (au niveau de laquelle s'étend la collerette 132) soit en regard de la deuxième face de la plaque support 12 (opposée à la première face). L'opérateur visse ensuite la pièce de fixation 13 sur le pion 1141 en utilisant un outil (non représenté) coopérant avec les lumières 1321 ménagées dans la collerette 131. Le vissage de la pièce de fixation 13 induit le placage de la collerette 132 contre la plaque support 12 en appui contre la butée 1146 de la borne de connexion 114 : l'unité ultrasonore 11, la plaque support 12 et la pièce de fixation sont alors solidaires. On obtient ainsi le dispositif implantable 1 illustré à la figure 1.

### 5. *Conclusions*

Le dispositif implantable 1 permet le traitement et/ou l'imagerie d'une zone cérébrale d'intérêt.

La présence d'une collerette 132 sur la pièce de fixation 13 permet :

- d'une part de protéger la carte électronique principale 1113 contre d'éventuels dommages que pourrait provoquer l'aiguille lors de son insertion sous la peau du crâne du patient,
- d'autre part de limiter la déformation de la plaque support 12 en répartissant la force appliquée par l'extrémité de l'aiguille sur une grande surface de la plaque support 12.

Le lecteur aura compris que de nombreuses modifications peuvent être apportées à l'invention décrite précédemment sans sortir matériellement des nouveaux enseignements et des avantages présentés ici.

Par exemple, dans les différents modes de réalisation décrit précédemment, la plaque support était constituée d'un métal. Il est bien évident pour l'homme du métier que la plaque support peut être constituée dans un autre matériau qu'un métal, comme par exemple :

- un matériau polymère tel que du polyéthylène, du polystyrène, de l'acrylique, du polyétheréthercétone (PEEK) ou du poly (méthacrylate de méthyle) (PMMA),
- un élastomère thermoplastique tel que du PEBAX.

De même dans les différents modes de réalisation décrits précédemment, l'unité ultrasonore 11, la plaque support 12 et la pièce de fixation 13 étaient assemblées ensemble par vissage de la pièce de fixation 13 sur le pion 1141 de l'unité ultrasonore 11. Il est bien évident que d'autres modes de fixation (collage, emmanchement en force, etc.) peuvent être prévus pour l'assemblage de la plaque support 12 sur l'unité ultrasonore 11.

## Revendications

1. Dispositif implantable (1) au niveau d'une ouverture ménagée dans la boîte crânienne d'un patient, le dispositif implantable (1) comprenant :

    - une plaque support (12) incluant une première face et une deuxième face opposée à la première face,
    - une unité ultrasonore (11) destinée à être montée sur la plaque support (12), ladite unité ultrasonore (11) comprenant :

        o un logement (111) incluant un fond (1111), au moins une paroi latérale (1112) et une face supérieure opposée au fond (1111), le logement (111) étant destiné à être positionné sur la première face de la plaque support (12),
        o une pluralité de transducteurs, disposés dans le logement (111), pour la génération d'ondes ultrasonores de traitement d'une affection cérébrale,
        o une borne de connexion électrique (114), fixée au logement (111), pour le raccordement de l'unité ultrasonore (11) à une unité de commande distante par l'intermédiaire de moyens de connexion électrique,

    - la borne de connexion (114) comprenant un pion (1141) s'étendant en saillie vers l'extérieur du logement (111) dans une direction perpendiculaire au fond (1111) et d'orientation allant du fond (1111) vers la face supérieure du logement (111),

    - la plaque support (12) comprenant un orifice traversant (122) pour le passage du pion (1141),

    **caractérisé en ce que:**

    - le dispositif implantable (1) comprend en outre une pièce de fixation (13) incluant :

        o un conduit (131) adapté pour recevoir au moins une portion du pion (1141), et
        o une collerette périphérique (132) s'étendant perpendiculairement à un axe longitudinal du conduit (131),

    la collerette (132) étant destinée à venir en contact avec la deuxième face de la plaque support (12) pour plaquer la plaque support (12) contre le logement (111) lorsque l'unité ultrasonore (11), la plaque support (12) et la pièce de fixation (13) sont assemblées.

2. Dispositif selon la revendication 1, *dans lequel* le rapport entre la largeur ($L_{Coll}$) de la collerette (132) et la largeur ($L_{Cond}$) du conduit (131) est compris entre ¼ et 2, préférentiellement ½ et $^3/_2$, et encore plus préférentiellement égal à 1.

3. Dispositif selon la revendication 1, *dans lequel* le rapport entre la largeur ($L_{Coll}$) de la collerette (132) et la largeur ($L_{Log}$) de la face supérieure du logement (111) est compris entre ½ et 2, et préférentiellement entre $^2/_3$ et 1.

4. Dispositif selon l'une quelconque des revendications 1 à 3, *dans lequel* la collerette (132) est de forme circulaire.

5. Dispositif selon l'une quelconque des revendications 1 à 4, *dans lequel* la collerette (132) comprend au moins deux lumières traversantes (1321) diamétralement opposées.

6. Dispositif selon l'une quelconque des revendications 1 à 5, *dans lequel* le pion (1141) est de forme cylindrique et comprend un filetage (1145) sur sa paroi latérale, le conduit (131) consistant en un écrou dont le trou taraudé est destiné à coopérer par vissage avec le filetage (1145) de la paroi latérale du pion (1141).

7. Dispositif implantable selon l'une quelconque des revendications 1 à 6, *dans lequel* le pion (1141) comprend une paroi supérieure (1142) opposée au fond (1111) du logement (111), et un trou borgne (1143) destiné à recevoir une extrémité des moyens de connexion, la paroi supérieure (1143) incluant une fraisure (1144) ménagée à l'entrée du trou borgne (1143).

8. Dispositif implantable selon l'une quelconque des revendications 1 à 7, *dans lequel* le pion (1141) comprend une butée (1146) à sa base, le bord de l'orifice traversant (122) de la plaque support (12) étant destiné à venir en appui contre la butée (1146) lorsque le pion (1141) est inséré dans ledit orifice traversant (122).

9. Dispositif implantable selon l'une quelconque des revendications 1 à 8, *dans lequel* le bord de l'orifice traversant (122) de la plaque support (12) est recouvert d'une couche (125) de matériau polymère, tel que de la silicone.

10. Dispositif implantable selon l'une quelconque des revendications 1 à 9, *dans lequel* la distance entre le centre de l'orifice traversant (122) et le barycentre (123) de la plaque (12) est non nulle.

11. Système pour l'imagerie et/ou le traitement d'un tissu cérébral, le système incluant une unité de commande et des moyens de connexion électrique, *caractérisé en ce que* le système comprend en outre un dispositif implantable (1) selon l'une quelconque des revendications 1 à 10.


**Patentansprüche**

1. Vorrichtung (1), die im Bereich einer Öffnung in der Schädelhöhle eines Patienten implantierbar ist, wobei die implantierbare Vorrichtung (1) umfasst:

   - eine Trägerplatte (12) mit einer ersten Seite und einer der ersten Seite gegenüberliegenden zweiten Seite,
   - eine Ultraschalleinheit (11), die zur Anbringung auf der Trägerplatte (12) vorgesehen ist, wobei die Ultraschalleinheit (11) umfasst:

      o eine Aufnahme (111) mit einem Boden (1111), mindestens einer Seitenwand (1112) und einer dem Boden (1111) gegenüberliegenden Oberseite, wobei die Aufnahme (111) zur Positionierung auf der ersten Seite der Trägerplatte (12) bestimmt ist,
      o eine Vielzahl von Wandlern, die in der Aufnahme (111) zur Erzeugung von Ultraschallwellen zur Behandlung einer Hirnerkrankung angeordnet sind,
      o eine an der Aufnahme (111) befestigte elektrische Anschlussklemme (114) zur Verbindung der Ultraschalleinheit (11) mit einer entfernten Steuereinheit über elektrische Verbindungsmittel,

   - wobei die Anschlussklemme (114) einen Stift (1141) umfasst, der aus der Aufnahme (111) in einer Richtung senkrecht zum Boden (1111) und in einer Ausrichtung vom Boden (1111) zur Oberseite der Aufnahme (111) herausragt,
   - wobei die Trägerplatte (12) eine Durchgangsöffnung (122) für den Durchgang des Stifts (1141) umfasst,

   **dadurch gekennzeichnet, dass**:

   - die implantierbare Vorrichtung (1) ferner ein Befestigungsteil (13) umfasst mit:

      o einem Kanal (131), der zum Empfang mindestens eines Abschnitts des Stifts (1141) geeignet ist, und
      o einem umlaufenden Flansch (132), der sich senkrecht zu einer Längsachse des Kanals (131) erstreckt,

   wobei der Flansch (132) dazu bestimmt ist, mit der zweiten Seite der Trägerplatte (12) in Kontakt zu kommen, um die Trägerplatte (12) gegen das Aufnahme (111) zu drücken, wenn die Ultraschalleinheit (11), die Trägerplatte (12) und das Befestigungsteil (13) zusammengebaut sind.

2. Vorrichtung nach Anspruch 1, wobei das Verhältnis zwischen der Breite ($L_{Coll}$) des Flansches (132) und der Breite ($L_{Cond}$) des Kanals (131) zwischen 1/4 und 2, vorzugsweise zwischen 1/2 und 3/2 liegt und noch bevorzugter gleich 1 ist.

3. Vorrichtung nach Anspruch 1, wobei das Verhältnis zwischen der Breite ($L_{Coll}$) des Flansches (132) und der Breite ($L_{Log}$) der Oberseite der Aufnahme (111) zwischen 1/2 und 2, vorzugsweise zwischen 2/3 und 1 liegt.

4. Vorrichtung nach einem der Ansprüche 1 bis 3, wobei der Flansch (132) kreisförmig ist.

5. Vorrichtung nach einem der Ansprüche 1 bis 4, wobei der Flansch (132) mindestens zwei diametral gegenüberliegende Durchgangslöcher (1321) aufweist.

6. Vorrichtung nach einem der Ansprüche 1 bis 5, wobei der Stift (1141) zylindrisch geformt ist und ein Gewinde (1145) an seiner Seitenwand aufweist, wobei der Kanal (131) aus einer Mutter besteht, deren Gewindebohrung dazu bestimmt ist, mit dem Gewinde (1145) an der Seitenwand des Stifts (1141) durch Schrauben zusammenzuwirken.

7. Implantierbare Vorrichtung nach einem der Ansprüche 1 bis 6, wobei der Stift (1141) eine dem Boden (1111) der Aufnahme (111) gegenüberliegende obere Wand (1142) und ein Sackloch (1143) umfasst, das zum Empfang eines Endes der Verbindungsmittel bestimmt ist, wobei die obere Wand (1143) eine Ausfräsung (1144) am Eingang des Sacklochs (1143) aufweist.

8. Implantierbare Vorrichtung nach einem der Ansprüche 1 bis 7, wobei der Stift (1141) an seiner Basis einen Anschlag (1146) aufweist, wobei der Rand der Durchgangsöffnung (122) der Trägerplatte (12) dazu bestimmt ist, am Anschlag (1146) anzuliegen, wenn der Stift (1141) in die Durchgangsöffnung (122) eingesetzt ist.

9. Implantierbare Vorrichtung nach einem der Ansprüche 1 bis 8, wobei der Rand der Durchgangsöffnung (122) der Trägerplatte (12) mit einer Schicht (125) aus Polymermaterial, wie beispielsweise Silikon, bedeckt ist.

10. Implantierbare Vorrichtung nach einem der Ansprüche 1 bis 9, wobei der Abstand zwischen der Mitte der Durchgangsöffnung (122) und dem Schwerpunkt (123) der Platte (12) ungleich Null ist.

11. System zur Bildgebung und/oder Behandlung von Hirngewebe, wobei das System eine Steuereinheit und elektrische Verbindungsmittel umfasst, **dadurch gekennzeichnet, dass** das System ferner eine implantierbare Vorrichtung (1) nach einem der Ansprüche 1 bis 10 umfasst.

**Claims**

1. An implantable device (1) at an aperture provided in the braincase of a patient, the implantable device (1) comprising:

    - a support plate (12) including a first face and a second face opposite the first face,
    - an ultrasonic unit (11) intended to be mounted on the support plate (12), said ultrasonic unit (11) comprising:

        o a housing (111) including a bottom (1111), at least one side wall (1112) and an upper face opposite the bottom (1111), the housing (111) being intended to be positioned on the first face of the support plate (12),
        o a plurality of transducers, disposed in the housing (111), for the generation of ultrasonic waves for treating a brain condition,
        o an electric connection terminal (114), attached to the housing (111), for connecting the ultrasonic unit (11) to a remote control unit via electrical connection means,

    - the connection terminal (114) comprising a pin (1141) projecting outwards from the housing (111) in a direction perpendicular to the bottom (1111) and oriented from the bottom (1111) towards the upper face of the housing (111),
    - the support plate (12) comprising a through-opening (122) for the pin (1141) to pass through,

**characterized in that:**

    - the implantable device (1) further comprises an attachment piece (13) including:

        o a conduit (131) adapted to receive at least a portion of the pin (1141), and
        o a peripheral collar (132) extending perpendicularly to a longitudinal axis of the conduit (131),

    the collar (132) being intended to contact the second face of the support plate (12) to press the support plate (12) against the housing (111) when the ultrasonic unit (11), the support plate (12) and the attachment piece (13) are joined.

2. The device according to claim *1, **wherein*** the ratio between the width ($L_{Coll}$) of the collar (132) and the width ($L_{Cond}$) of the conduit (131) is comprised between ¼ and 2, preferably ½ and $3/_2$, and even more preferably equal to 1.

3. The device according to claim 1, **wherein** the ratio between the width ($L_{Coll}$) of the collar (132) and the width ($L_{Log}$) of the upper face of the housing (111) is comprised between ½ and 2, and preferably between $2/_3$ and 1.

4. The device according to any one of claims 1 to 3, **wherein** the collar (132) is circular in shape.

5. The device according to any one of claims 1 to 4, **wherein** the collar (132) comprises at least two diametrically opposite through-lumens (1321).

6. The device according to any one of claims 1 to 5, **wherein** the pin (1141) is cylindrical in shape and comprises a threading (1145) on its side wall, the conduit (131) consisting of a nut the tapped hole of which is intended to cooperate by screwing with the threading (1145) of the side wall of the pin (1141).

7. The implantable device according to any one of claims 1 to 6, **wherein** the pin (1141) comprises an upper wall (1142) opposite the bottom (1111) of the housing (111), and a blind hole (1143) intended to receive one end of the connection means, the upper wall (1143) including a countersink (1144) made at the entrance to the blind hole (1143).

8. The implantable device according to any one of claims 1 to 7, *wherein* the pin (1141) comprises a stop (1146) at its base, the edge of the through-opening (122) of the support plate (12) being intended to bear against the stop (1146) when the pin (1141) is inserted into said through-opening (122).

9. The implantable device according to any one of claims 1 to 8, *wherein* the edge of the through-opening (122) of the support plate (12) is covered with a layer (125) of polymeric material, such as silicone.

10. The implantable device according to any one of claims 1 to 9, **wherein** the distance between the center of the through-opening (122) and the barycenter (123) of the plate (12) is non-zero.

11. A system for imaging and/or treatment of a brain tissue, the system including a control unit and electrical connection means, *characterized in that* the system further comprises an implantable device (1) according to any one of claims 1 to 10.

**FIG.1**

**FIG.2**

12

122

124

123

125

124

121

**FIG.3**

13

1312

131

132

L_Coll

L_Cond

1311

1321

FIG.4

**EP 4 065 222 B1**

**Documents brevets cités dans la description**

- FR 3078879 A1